# EUROPEAN PATENT APPLICATION

(11) **EP 1 865 072 A1**
(43) Date of publication of application: **12.12.2007**
(21) Application number: 06714783.5
(22) Date of filing: 27.02.2006
(51) Int. Cl.: C12Q 1/68, C12C 11/00, C12G 1/00, C12G 3/00, C12M 1/00, C12N 15/09

(54) **METHOD OF CONTROLLING THE FLAVOR OF ALCOHOLIC DRINK**

(30) Priority: 03.03.2005 JP 2005059716
(71) Applicant: Sapporo Breweries Limited, Tokyo 150-8522 (JP)
(72) Inventor: KOBAYASHI, Naoyuki, Sapporo Breweries Limited, Yaizu-shi, Shizuoka, 425-0013 (JP); SATO, Masahide, Sapporo Breweries Limited, Yaizu-shi, Shizuoka, 425-0013 (JP); FUKUHARA, Syunsuke, Kiyosu-shi, Aichi 452-0944 (JP); YOKOI, Shigehisa, c/o Sapporo Breweries Limited, Hita-shi, Oita 877-0054 (JP)
(74) Representative: Grünecker, Kinkeldey, Stockmair & Schwanhäusser Anwaltssozietät
(86) International application number: PCT/JP2006/303646
(87) International publication number: WO 2006/093104

(57) **Abstract**

It is an object of the present invention to provide a method for identifying brewer's yeast genes associated with alcoholic beverage flavor, to allow production of alcoholic beverages with desirable flavor by controlling the expression of the identified genes. In order to achieve this object, there is provided a method for identifying brewer's yeast genes using gene analysis means employing a DNA array or a DNA array and two-dimensional electrophoresis, a DNA array binding identified genes, and a method of selecting brewer's yeast using the DNA array.

## Description

### Technical Field

The present invention relates to a method for controlling alcoholic beverage flavor, and particularly it relates to a method and DNA array for identifying brewer's yeast genes, to a brewer's yeast selection method, to an alcoholic beverage production process and to alcoholic beverages.

### Background Art

For fermented foods and beverages obtained using yeast, flavor is a major quality-determining factor. For example, alcoholic beverages (beer, wine, sake and the like), fermented seasonings (miso, soy sauce, vinegar and the like) and breads have characteristics that are determined by the favoring components (esters, alcohols, etc.) in the yeast used.

Traditional methods of selecting useful brewer's yeasts such as beer yeast have been carried out by the methods used to select fine beer yeast used in beer breweries. Such methods have required taking a number of single cells from a yeast cell mass and then subjecting them to a fermentation test, analysis of flavor components by gas chromatography and a sensory test in order to select out the favorable yeast, and these procedures take considerable effort and time.

In recent years it has become possible to use genetic engineering techniques to introduce genes of interest into brewing genes and express them, and functional analysis of the genes and utilization of the analyzed genes permit breeding of yeast with desired features.

For example, the following yeast strains have had manipulation performed on genes relating to ester synthesis in beer yeast or sake yeast: a strain with increased isoamyl acetate production by high expression of the structural gene for an isoamyl acetate producing enzyme (ATF1) (Patent document 1); a strain with modified isoamyl alcohol, isobutyl alcohol and isoamyl acetate production by control (inhibition or promotion) of the expression of the structural gene for an amino group transferase acting on branched chain amino acids (Patent document 2); and a strain with increased isoamyl acetate production by disruption of an esterase gene that decomposes acetic acid esters (Patent document 3).

[Patent document 1] Japanese Unexamined Patent Publication HEI No. 6-062849
[Patent document 2] Japanese Unexamined Patent Publication HEI No. 11-235176
[Patent document 3] Japanese Unexamined Patent Publication HEI No. 9-234077

### Disclosure of the Invention

### Problems to be Solved by the Invention

However, the entire genomic nucleotide sequence of bottom fermentation yeast has not yet been public, unlike the baker's yeast *Saccharomyces cerevisiae,* whose entire genomic nucleotide sequence is already known, and very little has been determined regarding genes associated with the brewing properties of bottom fermentation yeast and the action of these genes in beer brewing. Furthermore, even when breeding brewer's yeast with an eye toward the differences in the expression level of a certain specific gene, the desired form of the gene can only be expressed by exhaustively analyzing the expression of several genes while determining that the gene does not vary due to other factors, and analysis of the expression of several genes at the same time in multiple specimens has been difficult to accomplish by conventional methods. In addition, even where genes associated with brewed alcohol flavor have been discovered, it has been difficult to exclude genes whose mRNA expression levels alone change without change in protein expression levels, in order to identify the genes that are positively correlated with both mRNA transcription levels and protein translation levels for controlling flavor.

It is therefore an object of the present invention to provide a method for identifying brewer's yeast genes associated with alcoholic beverage flavor, to allow production of alcoholic beverages with desirable flavor by controlling the alcoholic beverage flavor even in the absence of definitive information regarding the genes relating to brewing properties. It is another object of the invention to provide a method for selecting brewer's yeast with desirable flavor, and a DNA array to be used therefor.

### Means for Solving the Problems

In order to achieve the object stated above, the invention provides a method for identifying brewer's yeast genes associated with alcoholic beverage flavor, the method comprising (a) a step of using two different brewer's yeasts for fermentation under the same fermentation conditions, or using the same yeast for fermentation under two different fermentation conditions, to produce two different alcoholic beverages with differences in flavor, (b) a step of extracting the total RNA from the brewer's yeast during the fermentation in step (a), (c) a step of preparing two different cDNAs or two different mRNAs from the total RNA and labeling one cDNA or mRNA with a first fluorescent dye while labeling the other cDNA or mRNA with a second fluorescent dye to obtain two different labeled cDNAs or two different labeled mRNAs, (d) a step of competitive hybridization of the aforementioned two different labeled cDNAs or two different labeled mRNAs on a DNA array binding plasmids containing genomic DNA fragments of the same or different brewer's yeast as the aforementioned brewer's yeast, on a plurality of spots on a substrate, (e) a step of measuring the fluorescence emitted by the first and second fluorescent dyes at each of the spots, (f) a step of comparing the intensity of fluorescence emitted by the first fluorescent dye with the intensity of fluorescence emitted by the second fluorescent dye, and (g) a step of analyzing the sequences of genomic DNA fragments in the plasmids bound at spots where a difference in fluorescence intensity is observed.

Specifically, the invention provides a method for identifying a brewer's yeast gene associated with alcoholic beverage flavor, the method comprising a step of selecting a factor that can affect alcoholic beverage flavor from among brewer's yeast strains or fermentation conditions and carrying out fermentation based on the selected factor to obtain a pair of fermenting brewer's yeasts for comparative contrast, and a step of comparing the pair of fermenting brewer's yeasts and identifying a gene (G1) having different levels of mRNA expression, wherein identification of the G1 gene is accomplished by a method comprising (1) a step of extracting total RNA from both of the pair of fermenting brewer's yeasts, (2) a step of obtaining cDNA or cRNA labeled with a first fluorescent dye from one of the total RNAs and obtaining cDNA or cRNA labeled with a second fluorescent dye from the other of the total RNAs, (3) a step of competitive hybridization of the cDNA labeled with the first fluorescent dye and the cDNA labeled with the second fluorescent dye, or the cRNA labeled with the first fluorescent dye and the cRNA labeled with the second fluorescent dye, on a DNA array binding plasmids containing genomic DNA fragments of the same or different brewer's yeast as the aforementioned brewer's yeast, on a plurality of spots on a substrate, (4) a step of measuring the fluorescence emitted by the first fluorescent dye and the fluorescence emitted by the second fluorescent dye at each spot, (5) a step of comparing the intensity of fluorescence emitted by the first fluorescent dye with the intensity of fluorescence emitted by the second fluorescent dye, and (6) a step of identifying the G1 gene from the sequence of genomic DNA fragments in the plasmids bound at spots where a difference in fluorescence intensity is observed.

According to this gene identification method, expression of yeast genes during fermentation is compared with different yeast types and fermentation conditions, analyzing only the genes in which a difference is observed, and this eliminates the need to obtain nucleotide sequence information for the entire genome of the yeast beforehand. Also according to this method, it is possible to identify brewer's yeast genes associated with flavor, and to control the flavor of alcoholic beverages based on this information.

The invention still further provides a method for identifying a brewer's yeast gene associated with alcoholic beverage flavor, the method comprising a step of selecting a factor that can affect alcoholic beverage flavor from among brewer's yeast strains or fermentation conditions and carrying out fermentation based on the selected factor to obtain a pair of fermenting brewer's yeasts, a step of comparing the pair of fermenting brewer's yeasts and identifying genes (G1) having different levels of mRNA expression and genes (G2) having different levels of protein expression, and a step of selecting a common gene from among the identified G1 and G2 genes, wherein identification of the G1 gene is accomplished by a method comprising (1) a step of extracting total RNA from both of the pair of fermenting brewer's yeasts, (2) a step of obtaining cDNA or cRNA labeled with a first fluorescent dye from one of the total RNAs and obtaining cDNA or cRNA labeled with a second fluorescent dye from the other of the total RNAs, (3) a step of competitive hybridization of the cDNA labeled with the first fluorescent dye and the cDNA labeled with the second fluorescent dye, or the cRNA labeled with the first fluorescent dye and the cRNA labeled with the second fluorescent dye, on a DNA array binding plasmids containing genomic DNA fragments of the same or different brewer's yeast as the aforementioned brewer's yeast, on a plurality of spots on a substrate, (4) a step of measuring the fluorescence emitted by the first fluorescent dye and the fluorescence emitted by the second fluorescent dye at each spot, (5) a step of comparing the intensity of fluorescence emitted by the first fluorescent dye with the intensity of fluorescence emitted by the second fluorescent dye, and (6) a step of identifying the G1 gene from the sequence of genomic DNA fragments in the plasmids bound at spots where a difference in fluorescence intensity is observed, and wherein identification of the G2 genes is accomplished by a method comprising (i) a step of extracting crude protein from both of the pair of fermenting brewer's yeasts, (ii) a step of obtaining crude protein labeled with a third fluorescent dye from one of the crude proteins and crude protein labeled with a fourth fluorescent dye from the other of the crude proteins, (iii) a step of separating a mixture of the crude protein labeled with the third fluorescent dye and the crude protein labeled with the fourth fluorescent dye using two-dimensional electrophoresis, (iv) a step of measuring the fluorescence emitted by the third fluorescent dye and the fluorescence emitted by the fourth fluorescent dye binding to the fractionated protein, (v) a step of comparing the intensity of fluorescence emitted by the third fluorescent dye with the intensity of fluorescence emitted by the fourth fluorescent dye, and (vi) a step of analyzing the proteins exhibiting a difference in fluorescence intensity to identify G2 genes coding for the protein.

According to this gene identification method, it is possible to identify genes that are positively correlated with mRNA transcription levels and protein translation levels to control flavor, excluding the genes with only change in mRNA expression levels and no change in protein expression levels. Genes positively correlated with mRNA expression levels and protein expression levels allow the character of the brewer's yeast cell to be altered by artificially controlling only a single gene. Thus, after a gene coding for a protein associated with flavor has been identified, it can be used for easy and efficient control of alcoholic beverage flavor.

Preferred genomic DNA fragments of brewer's yeast to be included in the plasmids of the DNA array in step (3) are those derived from bottom beer yeast, the type most widely used for beer brewing.

The DNA array preferably further comprises another spot binding a brewer's yeast gene with a known function (hereinafter referred to as "known gene").

Using such a DNA array allows analysis of the sequence of a genomic DNA fragment at a spot exhibiting a pattern of fluorescence intensity (proportion of fluorescence intensity of a fluorescent dye) similar to or in synchrony with that of the spot binding the known gene, for identification of a gene present in a DNA fragment and inference of its function. For example, when the genomic DNA includes a gene that is functionally similar to a known gene important for brewing but that differs in its nucleotide sequence from the known gene, the gene can be identified by analyzing the sequence of a genomic DNA fragment whose difference in fluorescence intensity pattern, i.e. the state of gene expression of the two different yeasts, is similar to or in synchrony with the known gene. In other words, it is possible to discover a known or new gene whose expression pattern during fermentation is in synchrony with the known gene that is important for brewing and is functionally similar thereto, but has a different gene arrangement or amino acid sequence.

When the known gene is constitutively expressed regardless of the species or form of the brewer's yeast, a DNA array having the construction described above also allows easy evaluation of hybridization experiments at spots surrounding it (for example, in a block containing the spot), based on the fluorescence intensity at the spot of the known gene.

As a suitable type of DNA array comprising another spot binding the known gene, there may be used one wherein blocks consisting of a plurality of spots binding plasmids including brewer's yeast genomic DNA fragments and a spot binding the known gene, are arranged on a substrate. By using this type of DNA array, it is possible to infer functions of genes in genomic DNA fragments, or evaluate hybridization experiments, in each block with respect to the known gene. The type referred to above also includes a type wherein the blocks have equivalent shapes and spots binding the known gene are present at the same position of each block, which is particularly preferred since it facilitates screening of the positions of spots binding the plasmid or known gene.

The invention still further provides a DNA array wherein a gene associated with alcoholic beverage flavor that has been identified by the aforementioned method is bound at a plurality of spots on a substrate. The DNA array can be used to select brewer's yeasts based on flavor. Specifically, the invention provides a method for selecting brewer's yeasts based on flavor, the method comprising (a) a step of extracting total RNA from fermenting brewer's yeast, (b) a step of obtaining cDNA or cRNA labeled with a fluorescent dye from the aforementioned total RNA, (c) a step of hybridizing the fluorescent dye-labeled cDNA or cRNA onto the aforementioned DNA array, (d) a step of measuring the fluorescence emitted by the fluorescent dye at each spot of the DNA array and (e) a step of evaluating expression of the fermenting brewer's yeast gene based on the intensity of fluorescence of the fluorescent dye at each spot. According to this method, brewer's yeast with high intensity of fluorescence at spots containing genes associated with flavor can be selected in step (e) to select out yeast suitable for production of alcoholic beverages with desirable flavor.

The invention still further provides a process for production of alcoholic beverages using brewer's yeast selected by the aforementioned yeast selection method, and alcoholic beverages produced by the process.

### Effect of the Invention

According to the invention, it is possible to identify brewer's yeast genes associated with flavor, to select yeast with preferred brewing properties based on that information, and to produce alcoholic beverages with desirable flavor.

### Brief Description of the Drawings

Fig. 1 is a plan view schematically showing a DNA array according to a first mode.
Fig. 2 is a plan view schematically showing a DNA array according to a second mode.
Fig. 3 is a plan view schematically showing the DNA array prepared in Example 1.
Fig. 4 is a plan view schematically showing a magnified view of one block of the DNA array prepared in Example 1.
Fig. 5 is a graph showing time-dependent change in the number of floating yeast.
Fig. 6 is a chart showing the results of analysis of extracted total RNA purity with a bioanalyzer.
Fig. 7 is a photograph showing fluorescence detected for four blocks forming a row at the edge in the direction of the short side of the DNA array.
Fig. 8 is an illustration showing the results of clustering, based on the fluorescence pattern on the DNA array.
Fig. 9 is a schematic diagram showing the position of the SBc33P17 region in *Saccharomyces cerevisiae* genomic DNA.
Fig. 10 is a photograph showing the spots of proteins downregulated by addition of methionine, among spots of proteins separated by two-dimensional electrophoresis.

### Explanation of Symbols

1: substrate, 2: plasmid, 3: known gene, 4: block, 10: DNA array, 20: DNA array, 30: DNA array.

### Best Mode for Carrying Out the Invention

### (Method for identifying brewer's yeast genes associated with alcoholic beverage flavor)

The method for identifying brewer's yeast genes associated with alcoholic beverage flavor according to a first mode of the invention comprises a step of selecting a factor that can affect alcoholic beverage flavor from among brewer's yeast strains or fermentation conditions and carrying out fermentation based on the selected factor to obtain a pair of fermenting brewer's yeasts for comparative contrast, and a step of comparing the pair of fermenting brewer's yeasts and identifying a gene (G1) having different levels of mRNA expression.

The fermentation conditions that can affect alcoholic beverage flavor include type of medium, medium aeration rate, fermentation temperature and fermentation time, and these may be varied to produce alcoholic beverages with differences in flavor. Differences in flavor may be evaluated by taste testing of the beer product by panelists. Differences in flavor are, for example, differences in aroma, fruity odor, grain odor, diacetyl, acidity, sweetness, saltiness, bitterness and mouth feel.

Identification of the G1 gene is accomplished by a method comprising (1) a step of extracting total RNA from both of a pair of fermenting brewer's yeasts, (2) a step of obtaining cDNA or cRNA labeled with a first fluorescent dye from one of the total RNAs (hereunder, cDNA labeled with a fluorescent dye will also be referred to as "labeled cDNA", and cRNA labeled with a fluorescent dye will be referred to as "labeled cRNA"), and obtaining cDNA or cRNA labeled with a second fluorescent dye from the other of the total RNAs, (3) a step of competitive hybridization of the cDNA labeled with the first fluorescent dye and the cDNA labeled with the second fluorescent dye, or the cRNA labeled with the first fluorescent dye and the cRNA labeled with the second fluorescent dye, on a DNA array binding plasmids containing genomic DNA fragments of the same or different brewer's yeast as the aforementioned brewer's yeast, on a plurality of spots on a substrate, (4) a step of measuring the fluorescence emitted by the first fluorescent dye and the fluorescence emitted by the second fluorescent dye at each spot, (5) a step of comparing the intensity of fluorescence emitted by the first fluorescent dye with the intensity of fluorescence emitted by the second fluorescent dye, and (6) a step of identifying a G1 gene from the sequence of genomic DNA fragments in the plasmids bound at spots where a difference in fluorescence intensity is observed.

The extraction of total RNA in step (1) may be carried out by, for example, the hot phenol method (extraction of RNA while lysing the yeast in heated phenol and sodium dodecylsulfate (SDS)), but it is preferably carried out by freezing the yeast with liquid nitrogen to obtain high purity RNA of low solubility, and then crushing them with a crusher and extracting the RNA with an RNA extraction reagent. The crusher may be a Cryopress (product of Microtec Co., Ltd.) and the RNA extraction reagent may be Isogen^{™} (product of Nippon Gene Co., Ltd.).

The labeled cDNA or labeled cRNA in step (2) may be prepared, for example in the case of labeled cDNA, by annealing OligodT primer to total RNA extracted from the brewer's yeast and synthesizing cDNA using reverse transcriptase in the presence of fluorescent dye-bonded dNTP, and removing off the unreacted substrate with a spin column. The OligodT column may also be used for isolation and purification of polyA⁺RNA from the total RNA, and reverse transcription reaction.

The labeled cRNA may be prepared by using total RNA or OligodT primer comprising the T7 promoter sequence attached to polyA⁺RNA to synthesize unlabeled cDNA by reverse transcription reaction, reacting T7 RNA polymerase with the cDNA in the presence of fluorescent dye-bonded NTP to synthesize cRNA, and removing the unreacted substrate with a spin column.

The labeling used to obtain the labeled cDNA or labeled cRNA is preferably a cyanine dye, and more preferably either the first fluorescent dye or second fluorescent dye is Cy3 (green dye) while the other is Cy5 (red dye), in order to allow clear distinction of colors (fluorescent wavelengths) between the type of brewer's yeast and the fermentation conditions.

The DNA array used for step (3) will now be explained.

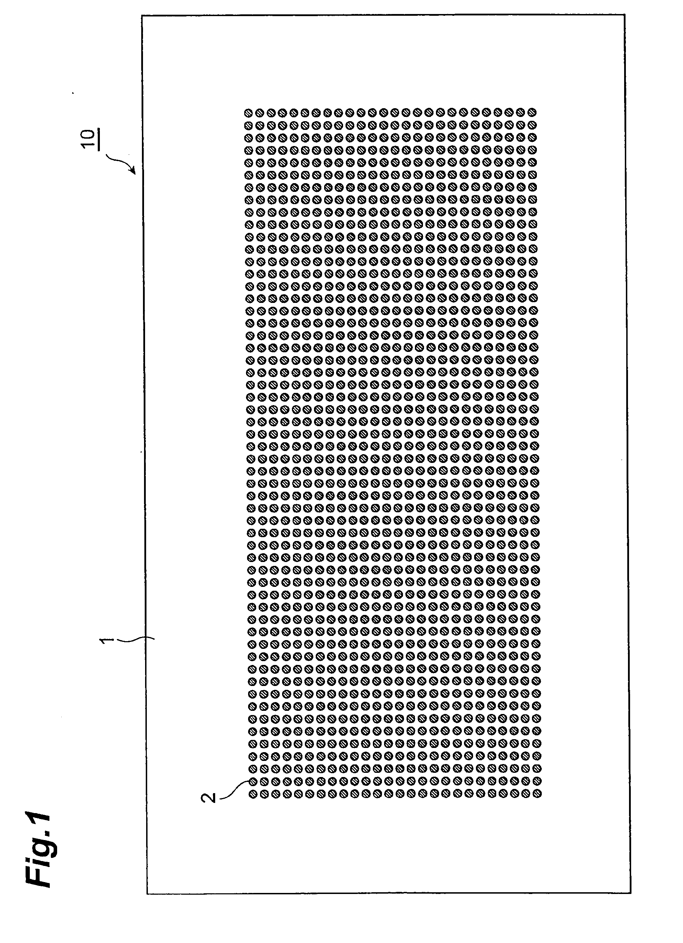

Fig. 1 is a plan view showing a DNA array according to a first mode. The DNA array 10 of the first mode is composed of a substrate 1 and plasmids 2 bound at a plurality of spots on the substrate 1. In the DNA array 10, the plurality of spots binding the plasmids 2 are arranged in a lattice fashion on the substrate 1.

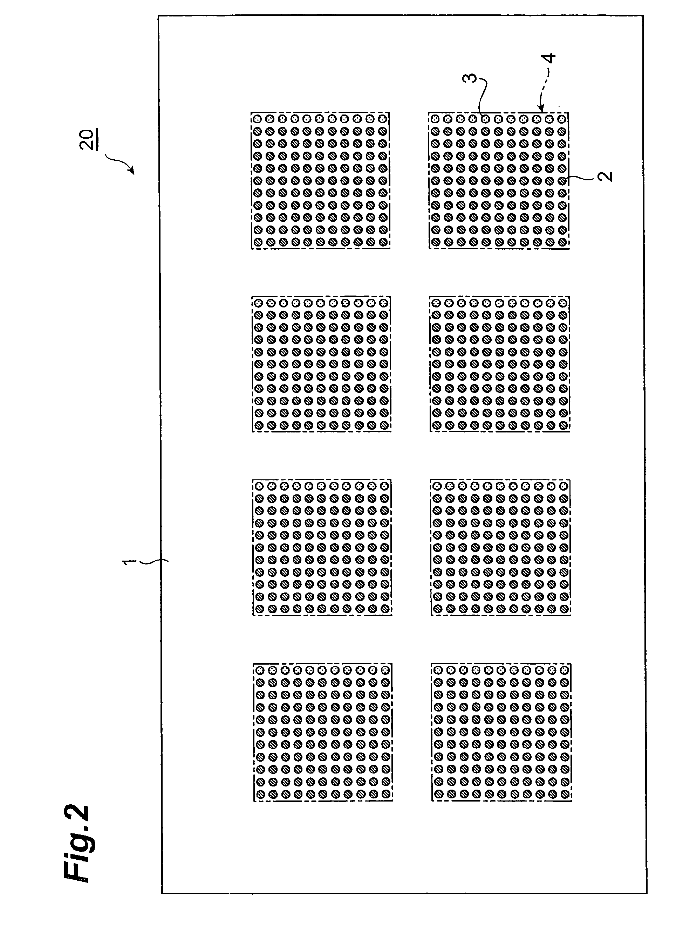

Fig. 2 is a plan view showing a DNA array according to a second mode. The DNA array 20 of the second mode comprises a substrate 1, plasmids 2 binding to a plurality of spots on the substrate 1, and a brewer's yeast gene 3 of known function binding to another spot on the substrate 1. In the DNA array 20, a plurality of blocks 4 composed of the plurality of spots binding the plasmids 2 and the spot binding the known gene 3 are arranged on the substrate 1. The plurality of blocks 4 have equivalent shapes, and the binding spot of the known gene 3 is at the same location of each block 4. The blocks 4 are composed of spots arranged in a lattice fashion, and the blocks 4 are also arranged in a lattice fashion on the substrate 1.

The material of the substrate 1 of the DNA array 10 or 20 may be any one that can stably bind the plasmids 2, and for example, there may be mentioned glass and synthetic resins (polycarbonate and other plastics). The substrate 1 is preferably in the form of a sheet or film.

The plasmids 2 include genomic DNA fragments of brewer's yeast. The brewer's yeast is preferably bottom beer yeast, and more preferably *Saccharomyces pastorianus Weihenstephan* 34/70 which is the most widely used yeast for beer brewing.

The sizes of the genomic DNA fragments in the plasmids 2 are preferably 1.5-3.0 kbp. If they are shorter than 1.5 kbp, the number of required genomic DNA fragments will tend to be increased when it is attempted to form a DNA array comprising the brewer's yeast genomic DNA in a comprehensive manner, and this will result in increased cost of preparing the DNA array. If they are longer than 3.0 kbp, on the other hand, there will tend to be more genes or exons in a single DNA fragment, making the process of gene expression analysis more complex.

The actual redundancy of the DNA array (the total length of genomic DNA fragments on the DNA array divided by the genome size of the brewer's yeast used to prepare the DNA array) is preferably between 1.5 and 2.0. An actual redundancy of less than 1.5 will tend to reduce the size of the region of the genome covered by the genomic DNA fragments on the DNA array, and if the actual redundancy is greater than 2.0, there will be larger regions of overlap between genomic DNA fragments on different spots, thus tending to complicate gene expression analysis.

Incidentally, brewer's yeast genomic DNA fragment inserts may be directly bonded to the DNA array instead of the plasmids 2. In this case as well, the sizes and redundancy of the genomic DNA fragments are preferably the same as when using the plasmids 2.

The known gene 3 composing the DNA array 20 can be selected by the experimenter according to the purpose. The known gene 3 is preferably a gene derived from *Saccharomyces cerevisiae,* whose sequence data can be obtained from the Saccharomyces Genome DataBase (http://www.yeastgenome.org/).

The plasmids 2 in the DNA array 20 preferably differ depending on the block. The same known gene 3 is preferably bonded at the same position of each block.

When a plurality of different known genes 3 are present on each block 4, at least one of them is preferably a constitutively expressed gene regardless of the species or form of the brewer's yeast. Having such a gene on each block will allow easier evaluation of hybridization experiments at each block. One such gene that may be mentioned is the ACT1 gene of *Saccharomyces cerevisiae.*

If a plurality of spots binding plasmids or known genes are arranged in a lattice fashion as in the DNA array 10 or 20, the spot location can be represented by coordinates, for easier identification of spot locations and more convenient gene expression analysis. In the DNA array 20, preferably the same known gene is bound at the same location of each spot.

A DNA array according to the invention is prepared by binding plasmids, randomly selected from a random genomic library of brewer's yeast, onto a substrate surface.

The random genomic library of brewer's yeast may be prepared by the following procedures 1)-6). Each procedure may be carried out by a publicly known method, unless otherwise specified.
1) The genomic DNA is extracted and purified from brewer's yeast.
2) The extracted and purified genomic DNA is fragmented. Fragmentation of the genomic DNA is preferably accomplished using a DNA fragmenting device in order to obtain random DNA fragments.
3) DNA fragments of a fixed size are recovered from the obtained genomic DNA fragments by electrophoresis.
4) The obtained DNA fragments are blunt ended and ligated to an appropriate plasmid vector.
5) The obtained recombinant vector is introduced into appropriate host cells.
6) The host cells are cultured on appropriate medium and transformed cells are selected.

Binding of the plasmid to the substrate surface can be accomplished by a method in which, for example, a fixing solution dissolving the genomic DNA fragment-containing plasmids is spotted on a surface-treated (for example, polylysine-treated) substrate with a spotter, and the plasmids are thus immobilized on the substrate surface. Using an alkaline fixing solution as the fixing solution will separate each of the double-stranded plasmids into two single-stranded plasmids in the fixing solution. When a non-alkaline fixing solution is used, the double strands may be converted to single strands by heat treatment or the like.

In the competitive hybridization in step (3), the labeled cDNA or labeled cRNA is denatured by heat treatment or the like before hybridization. The hybridization may be carried out, for example, by the method described in "DNA Arrays and State-of-the-Art PCR Methods" (Shujunsha Publishing).

Measurement of the fluorescence intensity in step (4) may be accomplished by acquiring image data with a CCD camera or scanner and digitizing it with software.

The comparison of the fluorescence intensities in step (5) can be accomplished by calculating the ratio between the fluorescence intensities of the first and second fluorescent dye, measured in step (4).

For sequence analysis of the DNA fragments in step (6), the nucleotide sequences of the genomic DNA fragments are determined, and then the genes in the DNA fragments are identified and the functions affecting alcoholic beverage flavor are predicted. Specifically, where differences in measured fluorescence intensities are observed, the nucleotide sequences of those genomic DNA fragments are determined, and these are associated with the strain and form of yeast, the genes are identified by database research (homology search or the like), and the function of the gene in each genomic DNA fragment is predicted. Detection of DNA fragments where differences in measured fluorescence intensities are observed is preferably accomplished by repeating steps (1)-(5) several times and clustering the results based on similarities in the expression ratio pattern. The sequence analysis may be carried out using a plasmid in the library corresponding to the genomic DNA fragment being analyzed.

When a DNA array 20 is used in step (3), the sequences of genomic DNA fragments exhibiting a pattern of fluorescence intensity (proportion of fluorescence intensity between the first and second fluorescent dye) similar to or in synchrony with the known gene are analyzed in step (4) for identification of genes present in the DNA fragments and inference of their function. In this case, the nucleotide sequences of the genes are determined, they are associated with the function of the known gene, and a database research (homology search or the like) is conducted to predict the function of the gene in each DNA fragment. DNA fragments exhibiting a pattern of fluorescence intensity similar to or in synchrony with the known gene are preferably detected by repeating steps (1)-(5) several times and clustering the results based on similarities in the expression ratio patterns.

According to the invention, a step of identifying the gene region of ribosomal RNA in the genomic DNA fragment on the DNA array is preferably carried out before reaching step (6). If the gene region of ribosomal RNA is identified, it will be possible to accomplish highly precise analysis by excluding the region from the sequence analysis.

The method for identifying brewer's yeast genes associated with alcoholic beverage flavor according to a second mode of the invention comprises a step of selecting a factor that can affect alcoholic beverage flavor from among brewer's yeast strains or fermentation conditions and carrying out fermentation based on the selected factor to obtain a pair of fermenting brewer's yeasts, a step of comparing the pair of fermenting brewer's yeasts and identifying genes (G1) having different levels of mRNA expression and genes (G2) having different levels of protein expression, and a step of selecting a common gene from among the identified G1 and G2 genes.

The method of identifying the fermentation conditions that affect alcoholic beverage flavor and the G1 genes are the same as for the first mode described above.

The step of selecting a common gene from among the identified G1 and G2 genes is a step in which common genes among G1 and G2 are identified and genes found in both groups are identified as brewer's yeast genes associated with alcoholic beverage flavor.

Identification of the G2 genes is accomplished by a method comprising (i) a step of extracting crude protein from both of the pair of fermenting brewer's yeasts, (ii) a step of obtaining crude protein labeled with a third fluorescent dye from one of the crude proteins and crude protein labeled with a fourth fluorescent dye from the other of the crude proteins, (iii) a step of separating a mixture of the crude protein labeled with the third fluorescent dye and the crude protein labeled with the fourth fluorescent dye using two-dimensional electrophoresis, (iv) a step of measuring the fluorescence emitted by the third fluorescent dye and the fluorescence emitted by the fourth fluorescent dye binding to the fractionated protein, (v) a step of comparing the intensity of fluorescence emitted by the third fluorescent dye with the intensity of fluorescence emitted by the fourth fluorescent dye, and (vi) a step of analyzing the proteins exhibiting a difference in fluorescence intensity to identify G2 genes coding for the protein.

The extraction of crude protein in step (i) is accomplished by adding a surfactant (SDS, Triton X, Tween-20 or the like), a reducing agent (DTT, 2-mercaptoethanol or the like), a protease inhibitor, urea and a cytolytic buffer containing Pharmalyte^{™} to yeast cells collected by centrifugal separation, and agitating the mixture for solubilization of the yeast cells. When solubilization is incomplete, the agitation may be followed by ultrasonic disruption of the yeast cells in an ice bath. After solubilization of the yeast cells, they are centrifuged and the supernatant is used as the crude protein extract.

The labeling with a fluorescent dye in step (ii) may be, for example, fluorescent labeling of the crude protein by adding a cyanine dye (Cy2, Cy3, Cy5) to the solubilized crude protein extract, stirring and incubating for a fixed period in an ice bath. The fluorescent dye may be any one whose color (fluorescent wavelength) can be distinguished for different yeast types or fermentation conditions, and either of the third fluorescent dye and fourth fluorescent dye is more preferably Cy3 (green dye) while the other is Cy5 (red dye).

The two-dimensional electrophoresis in step (iii) may be carried out according to a known method. Two-dimensional electrophoresis, comprising first-dimension separation by isoelectric electrophoresis followed by second-dimension separation by SDS-PAGE, is a sensitive method that allows detection based on minute differences in numbers and types of constituent amino acids. G2 is preferably identified by separating a mixture of the crude proteins labeled with two different fluorescent dyes using a gel having an isoelectric point of pH 4-7 and then performing electrophoresis with 12% SDS-polyacrylamide gel.

The fluorescence intensity in steps (iv) and (v) may be measured using an image analyzer equipped with a CCD camera and scanner, and the fluorescence intensity comparison in steps (iv) and (v) may be accomplished by digitizing the fluorescence intensities of the third and fourth fluorescent dyes at each spot of protein separated by the SDS-polyacrylamide gel after two-dimensional electrophoresis, and determining the ratio between them.

Analysis of the proteins in step (vi) may be done by extracting protein from each spot of the protein separated in the SDS-polyacrylamide gel after two-dimensional electrophoresis, and analyzing the obtained trace protein using a mass spectrometer, protein sequencer, amino acid analyzer or the like, although analysis with a mass spectrometer is preferred. Spots of protein in which a difference in expression is found are cut out from the gel and the trypsin-digested samples analyzed with a mass spectrometer, to allow the protein to be identified in a short period of time while also allowing identification of the gene coding for it.

### (DNA array)

The DNA array of the invention comprises a gene associated with alcoholic beverage flavor that has been identified by the aforementioned gene identification method, bound at a plurality of spots on a substrate. The process for preparing the DNA array was described above. Since the DNA array binds genes associated with flavor, it may be utilized for the brewer's yeast selection method of the invention as described below.

### (Method of selecting brewer's yeast based on flavor)

The method of selecting brewer's yeast based on flavor according to the invention comprises the following steps (a)-(e).
(a) A step of extracting total RNA from fermenting brewer's yeast.
(b) A step of obtaining cDNA or cRNA labeled with a fluorescent dye from the aforementioned total RNA.
(c) A step of hybridizing the fluorescent dye-labeled cDNA or cRNA onto the DNA array of the invention.
(d) A step of measuring the fluorescence emitted by the fluorescent dye at each spot of the DNA array.
(e) A step of evaluating expression of the fermenting brewer's yeast gene based on the intensity of fluorescence of the fluorescent dye at each spot.

The total RNA extraction, preparation of fluorescent labeled cDNA or cRNA, hybridization and fluorescence measurement in each step are carried out in the manner described above. The DNA array used in the selection method of the invention is as described above, having a gene associated with alcoholic beverage flavor bound at a plurality of spots on a substrate. Thus, measurement of the degree of hybridization between these genes and the cDNA from the yeast to be evaluated can be performed based on the fluorescence intensity of the fluorescent dye. Depending on the strength of hybridization (strength of fluorescence intensity), it is possible to evaluate expression of the gene associated with the flavor of the yeast that is being evaluated, and to select yeast that produce a flavor desirable for alcoholic beverages based on the results of the evaluation.

### (Method of selecting brewer's yeasts that produce alcoholic beverages with desired flavor)

The following method of selecting brewer's yeast may also be employed as an alternative method for the aforementioned method of selecting the brewer's yeast. Specifically, the method of selecting brewer's yeasts that produce alcoholic beverages with desired flavor according to the invention comprises the following steps (a)-(c).
(a) A step of obtaining the following three different labeled cDNAs or three different labeled cRNAs.
   (a1) cDNA or cRNA labeled with a first fluorescent dye, synthesized from total RNA obtained by extraction of total RNA from fermenting brewer's yeast that produces an alcoholic beverage with the desired flavor.
   (a2) cDNA or cRNA labeled with a second fluorescent dye, synthesized from total RNA obtained by extraction of total RNA from fermenting brewer's yeast that produces an alcoholic beverage with a different flavor from the desired flavor.
   (a3) cDNA or cRNA labeled with a third fluorescent dye, synthesized from total RNA obtained by extraction of total RNA from a fermenting brewer's yeast that is to be evaluated.
(b) A step of separate competitive hybridization with the following two combinations on a DNA array binding plasmids containing genomic DNA fragments of a standard brewer's yeast on a plurality of spots on a substrate, and measurement of the difference in intensity of fluorescence emitted by each fluorescent dye at each spot.
   (b1) Combination of a first labeled cDNA and third labeled cDNA, or a combination of a first labeled cRNA and third labeled cRNA.
   (b2) Combination of a second labeled cDNA and third labeled cDNA, or a combination of a second labeled cRNA and third labeled cRNA.
(c) A step of judging that brewer's yeast wherein the difference in fluorescence intensity of (b1) is smaller than the fluorescence intensity of (b2) is a brewer's yeast that is more similar to brewer's yeast that produces alcohol with the desired flavor.

The total RNA extraction, preparation of fluorescent labeled cDNA or cRNA, DNA array, hybridization and fluorescence measurement in each step are carried out in the manner described above.

The yeast used for (a1) and (a2) are yeast whose alcoholic beverages fermented under the same conditions have been tested by panelists, and that are already known to produce alcoholic beverages with a particular flavor. The fluorescent dyes used to label (a1), (a2) and (a3) may all be different, or the fluorescent dyes used to label (a1) and (a2) may be the same.

It is believed that evaluated yeast with a smaller difference in fluorescence intensity of (b1) compared to the difference in fluorescence intensity of (b2) will exhibit a gene expression pattern similar to yeast that produces alcoholic beverages with the desired flavor, and therefore such yeast is selected as brewer's yeast that produces an alcoholic beverage with the desired flavor.

### (Alcoholic beverage production process and alcoholic beverages)

The process for production of alcoholic beverages according to the invention is characterized by using brewer's yeast selected by the method described above, and alcoholic beverages produced by the process are also encompassed by the invention.

The brewer's yeast selected by the aforementioned method is yeast with desirable brewing properties, and when the yeast is used to produce alcoholic beverages, alcoholic beverages with desirable flavor are obtained.

There are no particular restrictions on the process for producing alcoholic beverages, and an ordinary alcoholic beverage production process may be used with the only stipulation that the yeast used is a brewer's yeast selected by the method of the invention. For example, beer production processes normally include a mashing step, fermenting step and filtering step. In the mashing step, the malt, secondary materials and mashing water are mixed, the resulting mixture is heated for saccharification of the malt and secondary materials, and the sweet wort is obtained from the saccharified malt and secondary materials. In the fermenting step, yeast is added to the sweet wort obtained from the mashing step for fermentation, to obtain an intermediate malt alcoholic beverage product. Brewer's yeast selected by the method of the invention is used for the fermenting step. In the filtering step, the intermediate beer product obtained from the fermenting step is filtered to obtain beer. The filtering step is not essential, however.

The alcoholic beverage is preferably beer or other liquor. Beer is an alcoholic beverage produced by fermentation of malt, and there is no limitation on the proportion of malt material used or the alcohol content. That is, low-malt beer having a malt use percentage of less than 66.7% is also included as "beer". Other types of liquor are alcoholic beverages obtained by similar production processes as beer without using malt as the starting material, and as examples there may be mentioned alcoholic beverages obtained using pea protein instead of malt.

### Examples

Examples of the invention will now be explained. However, the invention is in no way limited to these examples.

### (Example 1: Preparation of DNA array)

### (1) Preparation of random genomic library:

A random genomic library for brewer's yeast was prepared using *Saccharomyces pastorianus Weihenstephan* 34/70 as the brewer's yeast.

Extraction and purification of genomic DNA from the brewer's yeast was carried out by a publicly known method, such as the one described in Methods in Yeast Genetics, A Cold Spring Harbor Laboratory Course Manual (Sadakazu Oya, ed., Maruzen Publishing, p.113-119).

The random genomic library was prepared in the following manner. 1) The genomic DNA was fragmented using a DNA fragmenting device (HydroShear). 2) The obtained DNA fragments were subjected to electrophoresis in agarose gel and an approximately 2.5 kbp DNA fragment was cut out. 3) The DNA fragment ends were blunted and then ligated in pUC19 vector. 4) The obtained recombinant vector was introduced into *E*. *coli* and glycerol stock was prepared from the resulting colonies. 5) The E. coli was transferred from the glycerol stock to LB medium (10 g/L bactotryptone, 5 g/L yeast extract, 10 g/L sodium chloride, pH 7.0) (liquid medium), and approximately 20,000 plasmid clones were extracted. Since each plasmid clone contains an approximately 2.5 kbp genomic DNA fragment, the entirety of the extracted plasmid clones includes a genomic DNA fragment of approximately 50 Mbp (2.5 kbp × 20,000 clones), and therefore it was estimated to have about 2.0 redundancy of the bottom beer yeast genome size of 24-26 Mbp.

### (2) Evaluation of random genomic library:

The random genomic library was evaluated in the following manner. 1) Plasmid DNA was digested with restriction enzyme BamHI and then subjected to agarose gel electrophoresis. 2) Base sequence analysis was then performed with vector primers, using as template plasmid DNA dispensed in eight 96-well plates. As a result, approximately 90% of the clones were confirmed to have genomic DNA inserts. That is, the actual redundancy of the prepared DNA array was estimated to be about 1.8 (2.0 × 0.9).

### (3) Preparation of DNA array:

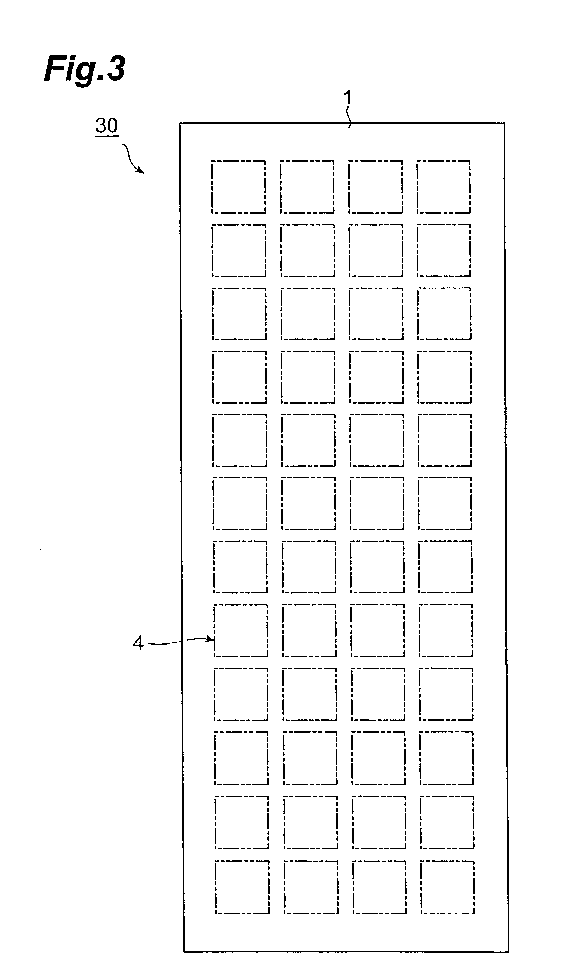

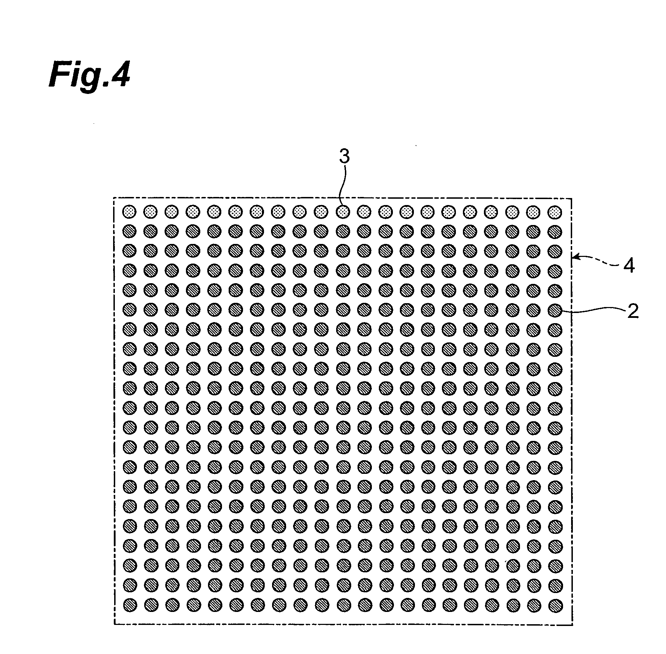

Fig. 3 is a plan view schematically showing the DNA array prepared in Example 1. As shown in Fig. 3, the DNA array 30 prepared in Example 1 was composed of blocks 4 with 12 × 4 rows (48) arranged on the substrate 1. Fig. 4 is a plan view schematically showing a magnified view of one block 4 of the DNA array 30. The block 4 is composed of 21 × 21 spots binding plasmids 2 or a known gene 3. The 21 spots binding the known gene 3 form a row at the edge of each block 4 in the direction of the short side of the DNA array 30. In Fig. 4, the blank is shown as the known gene 3 for convenience. Alternatively, different known genes 3 may be bound to different spots.

The DNA array 30 was prepared by immobilizing plasmids 2 on the spots on rows 2-21 of each block 4, and the known gene 3 on the first row spots of each block 4. A glass panel was used as the substrate 1, and the immobilization was accomplished by spotting the plasmids 2 or known gene 3 dissolved in an alkali-containing fixing solution on the glass panel after surface treatment (polylysine treatment).

A *Saccharomyces cerevisiae* gene was used as the known gene 3, and the DNA amplified by PCR was fixed. The names, functions and PCR amplification primers for the known genes fixed on the DNA array are listed as 1)-10) below.
1) Gene name: ACT 1, Function: actin synthesis, primers:
   (5'-TCGGTAGACCAAGACACCAA-3',
   5'-CCTTACGGACATCGACATCA-3')
2) Gene name: ATF1, Function: acetic acid ester synthesis, primers:
   (5'-GCCACATCCAGTGCATGATT-3',
   5'-TAGTTGTGAGCGGCAATCTG-3')
3) Gene name: ATF2, Function: acetic acid ester synthesis, primers:
   (5'-CGAAGAGGCCTAATTGGAGA-3',
   5'-TCACCGTTGTCGTACGATTC-3')
4) Gene name: Lg-ATF1, Function: acetic acid ester synthesis, primers:
   (5'-GGTGTGATTCTCAACGAGCA-3',
   5'-AACGGAGTGATGGTGCACTT-3')
5) Gene name: EHT1, Function: lipid metabolism, primers:
   (5'-TACCAGAGGTTGTGCACGTT-3',
   5'-TCTGCAATTGCCTTGGTAGC-3')
6) Gene name: IAH1, Function: esterase activity, primers:
   (5'-GATCAGTATGCTCTTGGAGC-3',
   5'-GTTGTTGCCAAGCATCACCA-3')
7) Gene name: LEU4, Function: isopropyl malate synthesis, primers:
   (5'-ACGGTGGAAGCATTAACAGG-3',
   5'-GGATCCAATGGCAAGTATGG-3')
8) Gene name: OLE1, Function: fatty acid desaturation, primers:
   (5'-CTCCGTTTTCTACTACGCTG-3',
   5'-GTTGGGTCGTATTGGTACCA-3')
9) Gene name: SSU1, Function: sulfite transport, primers:
   (5'-TGCTCTTACGAGGCAGTTTG-3',
   5'-ATGGCATGCAGCCACGTTAA-3')
10) Gene name: Lg-FLO1, Function: aggregating gene, primers:
   (5'-CAACAAAGCAAACCAAGGGG-3',
   5'-TTACCATACGATTGCCAGCA-3')

On each block 4, at the spots forming the row at the edge in the direction of the short side of the DNA array 30, 1: ACT1, 2: ATF1, 3: ATF2, 4: Lg-ATF1, 5: EHT1, 6: IAH1, 7: LEU4, 8: OLE1, 9-18: blank, 19: pUC19 (control), 20: SSU1, 21: Lg-FLO1 were fixed in order. That is, there were immobilized on the glass substrate surface of the DNA array 30, 528 (11×4 rows × 12 columns) known genes (including a control) and 20,160 (20 × 21 × 4 rows × 12 columns) of plasmids. The ACT1 gene is a gene constitutively expressed regardless of the strain or form of the brewer's yeast.

### (4) Identification of ribosomal RNA gene regions:

In order to identify the ribosomal RNA gene regions overlapping on the genomic DNA fragments on the DNA array, DNA fragments having the sequences shown below were used as primers for the 18S ribosomal RNA gene and 28S ribosomal RNA gene, with bottom beer yeast genomic DNA as template, for PCR amplification of complementary DNA fragments for the 18S ribosomal RNA gene region and 28S ribosomal RNA gene region among the genomic DNA of the bottom beer yeast.
1) Primers for 18S ribosomal RNA gene:
   (5'-CTGGTTGATYCTGCCAGT-3', 5'-CYGCAGGTTCACCTACRG-3')
2) Primers for 28S ribosomal RNA gene:
   (5'-RCATCGATGAAGAACGYWG-3', 5'-MRGGCTKAATCTCARYRGATCG-3')

The obtained DNA fragments were labeled with Cy5 for use as probes in hybridization on the DNA array. The hybridization was conducted according to a known method (for example, the method described in "DNA Arrays and State-of-the-Art PCR Methods" (Shujunsha Publishing), for 14 hours in a thermostatic bath at 62°C.

As a result, of the 20,160 DNA fragments fixed on the substrate, 1129 (approximately 6%) of the DNA fragments were judged to be ribosomal RNA gene regions and were masked in the subsequent analysis.

(Example 2: Gene expression analysis of bottom beer yeast during fermentation)
(1) Extraction of total RNA:
   A fermentation test using bottom beer yeast was conducted under the following conditions.
      · Sweet wort extract concentration: approximately 11 %
      · Sweet wort volume: 2.5 L
      · Sweet wort dissolved oxygen concentration: approximately 5-10 ppm
      · Fermentation temperature: approximately 13°C
      · Yeast input: 10-12 g wet yeast cells
      · Number of fermentations: 3

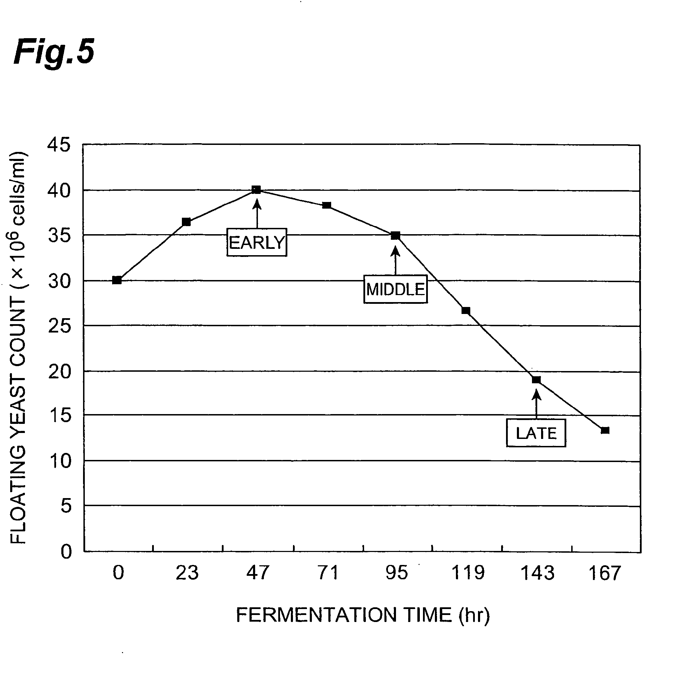

The fermentate was periodically sampled, and the time-dependent change in floating yeast count (yeast proliferation) was examined. Floating yeast was sampled at the early, middle and late stages of fermentation. Specifically, sampling was performed at 47, 95 and 143 hours from the start of fermentation. Fig. 5 is a graph showing the time-dependent change in the number of floating yeast.

Total RNA was extracted in the following manner from yeast at each of the three fermentation points. 1) Yeasts (approximately 1 g) collected from the fermentates were frozen with liquid nitrogen in centrifugation tubes. 2) The frozen yeast cells were crushed with a Cryopress (Microtec Co., Ltd.) (crushing three times for 30 seconds). 3) The crushate was lysed with approximately 10 ml of Isogen^{™} (Nippon Gene Co., Ltd.) and the total RNA was purified according to the manufacturer's manual.

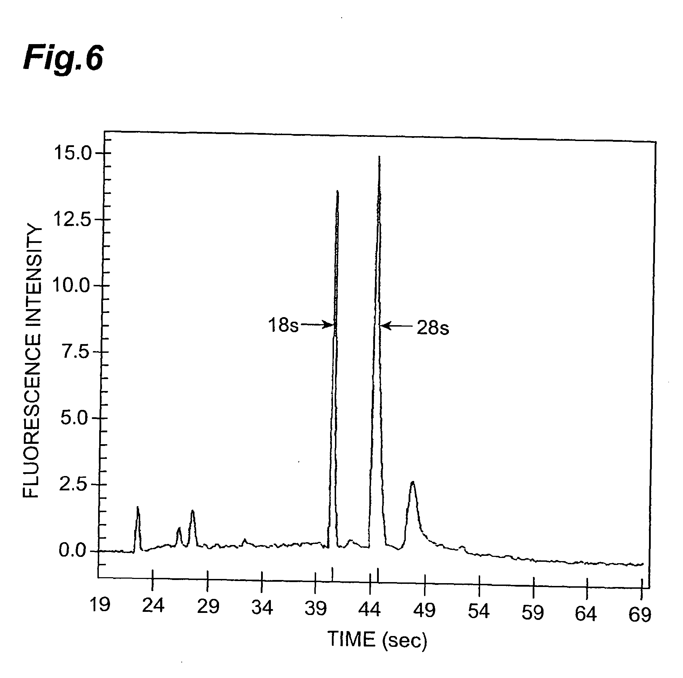

The concentration of total RNA extracted from the late-stage fermentation yeast was measured by spectrophotometry and the quality was confirmed using a bioanalyzer (Agilent 2100 Bioanalyzer). The concentration was 231.79 ng/µL. Fig. 6 is a chart showing the results of analysis of extracted total RNA purity with a bioanalyzer. As shown in Fig. 6, the proportion of 18S and 28S ribosomal RNA in the extracted total RNA was 1:1.6, and no 28S ribosomal RNA decomposition product was detected. The results indicated that highly pure total RNA had been extracted.

### (2) Labeling of fluorescent dyes:

After annealing OligodT primer to the total RNA, reverse transcriptase reaction was conducted in the presence of aminoallyl-dUTP, to prepare cDNA containing aminoallyl-dUTP. Coupling reaction was carried out to label the cDNA with cyanine dye (Cy3 or Cy5). One of the two cDNAs hybridized to the plasmids and known gene on each DNA array was labeled with Cy3 (green dye), while the other was labeled with Cy5 (red dye).

### Hybridization:

For each DNA array, the two fluorescent dye labeled cDNAs were competitively hybridized to the plasmids and known gene on the DNA array. The hybridization was conducted according to a known method (for example, the method described in "DNA Arrays and State-of-the-Art PCR Methods" (Shujunsha Publishing), for 14 hours in a thermostatic bath at 62°C.

### (3) Measurement of fluorescence intensity:

Following hybridization, the DNA array was washed and the signal was read with a slide scanner (Agilent Scanner, Agilent). The image data for the obtained signal were converted to numerical data using the proprietary software FeatureExtraction (Agilent). The fluorescence of the negative control was used as the background, and was subtracted from the fluorescence for each spot. Normalization between the sample and control (pUC19) was accomplished by the global normalization method (normalization using all spots).

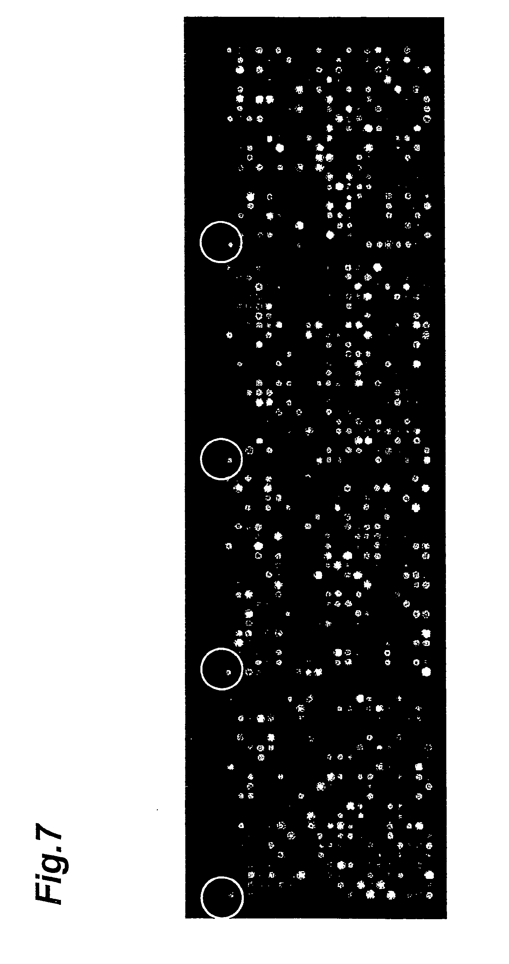

The results of the hybridization experiment were judged from the fluorescence intensity at 48 spots (4 rows × 12 columns) for the ACT1 gene in the scanned image. Fig. 7 is a photograph showing fluorescence detected for four blocks forming a row at the edge in the direction of the short side of the DNA array. In Fig. 7, spots binding the known gene (including blanks) compose a row at the edge on the short side of the DNA array, in each block. As shown in Fig. 7, the fluorescence intensity of the constitutively expressed ACT1 gene (indicated by white circles in the photograph) was approximately equivalent for each block, and therefore the results of the hybridization experiment were judged to be positive.

### (4) Sequence analysis:

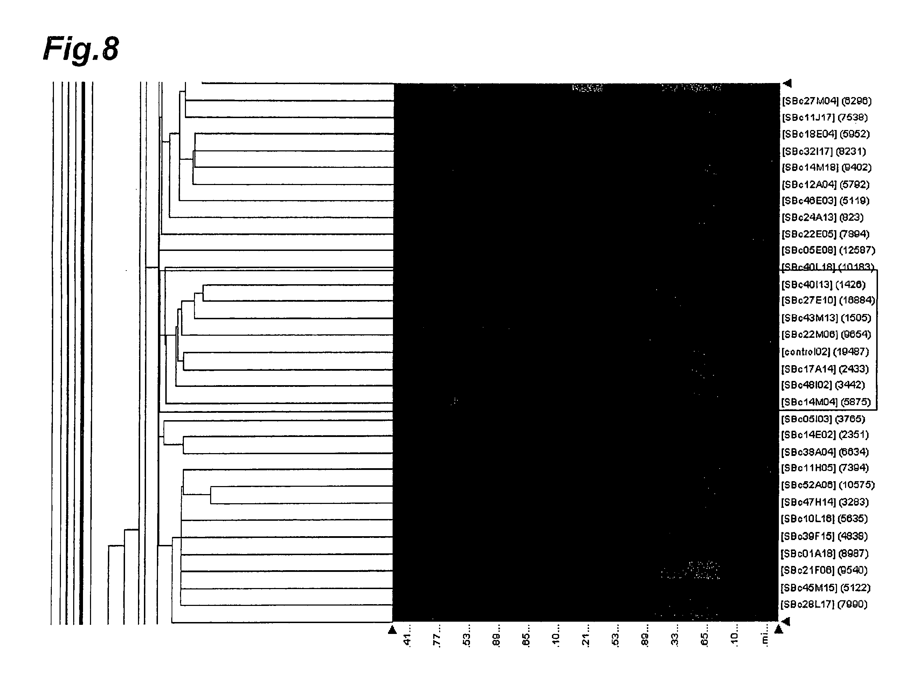

Clustering was performed based on fluorescence patterns on the DNA array obtained in three fermentation experiments. As the known genes there were selected seven spots whose fluorescence patterns were in synchrony with that of the spot fixing the ATF 1 gene coding for alcohol acetyltransferase. For each of these spots, the sequences of the corresponding genomic DNA fragments in the random genomic library were analyzed and the functions of the genes were inferred from a database search using BLAST or the like. As a result, the ten genes listed in Table 1 were annotated from six genomic DNA fragments (SBc40I13, SBc48I02, SBc14M04, SBc43M13, SBc27E10 and SBc22M06), and one of the genomic DNA fragments (SBc17A14) was a non*-Saccharomyces cerevisiae* type. Fig. 8 is an illustration showing the results of clustering, based on the fluorescence pattern on the DNA array. In Fig. 8, control 02 represents the ATF 1 gene.

**[Table 1]**

| | | | | | |
|---|---|---|---|---|---|
| CDC48 | YEL020C | SAP4 | PGM1 | YPK1 | NFI1 |
| PUP1 | PET123 | YOR376W-A | ATF1 | | |

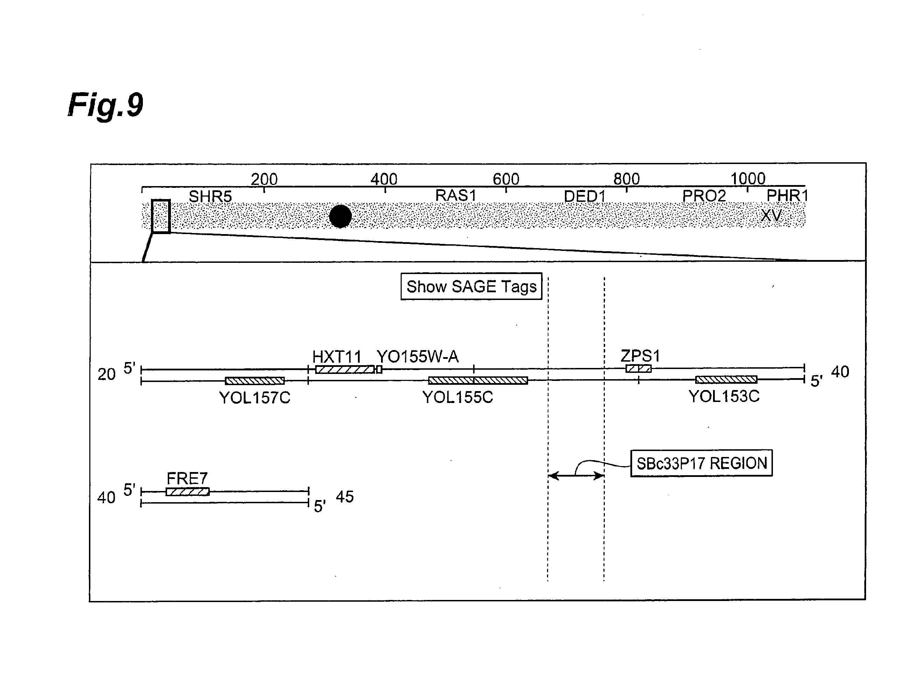

As a result of clustering, five spots were selected that had increasing gene expression during the fermentation process. Sequence analysis showed that the five genomic DNA fragments (SBc25J18, SBc35O20, SBc42N04, SBc32N16 and SBc33P17) corresponding to these spots were genomic DNA fragments equivalent to *Saccharomyces cerevisiae.* Fig. 9 is a schematic diagram showing the position of the SBc33P17 region in *Saccharomyces cerevisiae* genomic DNA. As shown in Fig. 9, SBc33P17 corresponds to a region of 32250 bp-34492 bp on chromosome XV of *Saccharomyces cerevisiae,* but this region does not include an open reading frame and is therefore a non-coding region.

(Example 3: Identification of gene associated with beer flavor expressed during fermentation of bottom beer yeast, by fluorescent labeling two-dimensional difference gel electrophoresis (2D-DIGE))

When methionine is added to bottom beer yeast for fermentation, the amount of hydrogen sulfide produced by the yeast is reduced, yielding a desirable beer flavor. Analysis was attempted of a protein whose expression level increases upon addition of methionine during fermentation of bottom beer yeast, by fluorescent labeling two-dimensional difference electrophoresis (2D-DIGE), to identify a brewer's yeast gene associated with beer flavor.

### (1) Extraction of crude protein:

A fermentation experiment using bottom beer yeast with and without addition of methionine (10 ppm) was conducted under the following conditions.
· Sweet wort extract concentration: approximately 11 %
· Sweet wort volume: 2.5 L
· Sweet wort dissolved oxygen concentration: approximately 5-10 ppm
· Fermentation temperature: 15°C
· Yeast input: 20-24 g wet yeast cells

When 23 hours had elapsed from the start of fermentation, the yeast cells used for fermentation with and without addition of methionine (10 ppm) were collected and used for the following crude protein extraction. First, 200 µL of solubilization buffer (7 M urea, 2M thiourea, 4% CHAPS, 1% DTT, 0.5% Pharmalyte^{™}) was added to 10 mg (dry weight) of the recovered yeast cells, and after stirring at room temperature for 5 minutes (vortex), the yeast cell were subjected to ultrasonic disruption (1 second × 10 times) in an ice bath. After shaking at room temperature for 30 minutes, centrifugation was performed (5 minutes × 10,000 g) and the protein solubilized fraction of the supernatant was used as the crude protein. The protein content of the supernatant was quantified by the Bradford method.

### (2) Preparation of two-dimensional electrophoresis sample (fluorescent labeling of crude protein)

To each solubilized crude protein (50 µg) there was added 400 pmol of the fluorescent dye CyDye DIGE fluors (Amersham Biosciences Corp.) developed for DIGE, and the mixture was stirred for 30 minutes of reaction in an ice bath for fluorescent labeling.

CyDye DIGE fluors contains three dyes with different excitation/fluorescent wavelengths (Cy2, Cy3, Cy5), which are designed to have equal molecular weights and charges. Therefore, a given protein labeled with any of these fluorescent dyes will have the same gel mobility in two-dimensional electrophoresis.

In this experiment, the crude protein of the fermented group with 10 ppm of methionine added was labeled with Cy3, and the crude protein of the fermented group without addition of methionine was labeled with Cy5. The labeling reaction was suspended by addition of 1 µL of L-lysine and reaction for 10 minutes in an ice bath. The two labeled crude proteins obtained in this manner were mixed in equal amounts and the labeled crude protein mixture sample was subjected to simultaneous two-dimensional electrophoresis in the same gel.

### (2) Two-dimensional electrophoresis

The two-dimensional electrophoresis was repeated three times under the following conditions. The first dimension of the two-dimensional electrophoresis employed a fixed pH (pH 4-7) gradient isoelectric focusing gel (hereinafter referred to as isoelectric focusing gel; Amersham Biosciences Corp.), for fractionation of 150 µg of the labeled crude protein mixture sample with a two-dimensional electrophoresis system (multiphor II: Amersham Biosciences Corp.).

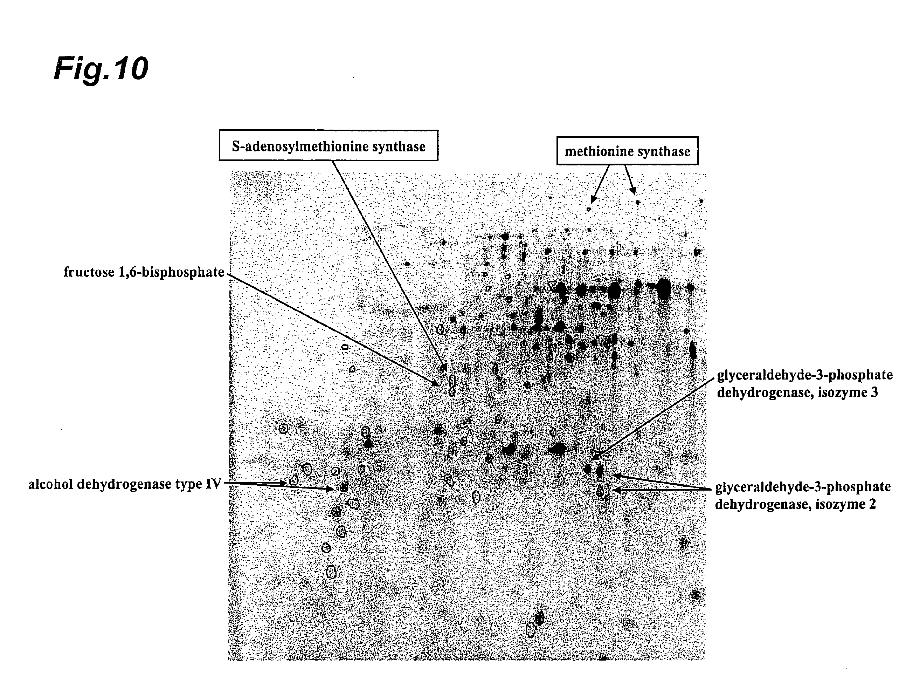

After the first dimension electrophoresis, the isoelectric focusing gel was equilibrated with DTT and then acetylated with iodoacetamide and SDS and used for second dimension electrophoresis. The second dimension electrophoresis was carried out at 2100 Vh using 12% SDS-polyacrylamide gel. Immediately upon completion of the electrophoresis, the gel was scanned with an image analyzer (Typhoon9400; Amersham Biosciences Corp.), and proteins whose expression was affected by addition or no addition of methionine were analyzed with specialized software. Fig. 10 is a photograph showing spots of proteins fractionated by two-dimensional electrophoresis, where the spots indicated by arrows are protein that were downregulated by addition of methionine.

### (3) Analysis of proteins downregulated by addition of methionine.

Of the regions (spots) of the gel where the proteins fractionated by two-dimensional electrophoresis emitted fluorescence, special consideration was given to spots where the fluorescence intensity of Cy3 (label of fermentation group with addition of 10 ppm of methionine) was significantly reduced compared to the fluorescence intensity of Cy5 (label of fermentation group with no addition of methionine). Judgment of a statistically significant difference was based on the following conditions: i) standard deviation of less than 30% between the spots of the samples (test repeated 3 times), ii) significant difference of p < 5% in a t-test
, iii) spot intensity proportion of less than 0.67% with respect to the control.

The spots with significantly reduced Cy3 fluorescence intensity compared to Cy5 fluorescence intensity were cut out as gel fragments from the fluorescence intensity-analyzed gel, digested with trypsin and then measured by mass spectrometry, after which a Mascot (http://www.Matrixscience.com) search was conducted against *Saccharomyces cerevisiae* to identify the protein. Table 2 shows proteins exhibiting statistically significant downregulation with addition of methionine.

**[Table 2]**

| Protein name | Gene name | Synonym |
|---|---|---|
| Methionine synthase | MET6 | YER091C |
| S-Adenosylmethionine synthase | SAM1 | YLR180W |

As a result, the two proteins methionine synthase and S-adenosylmethionine synthase (involved in methionine metabolism) were identified. These results suggested that expression of the MET6 gene is followed by production of two isozymes by post-translational modification. This phenomenon has not been reported for baker's yeast (*Saccharomyces cerevisiae*), and is specific to brewer's yeast.

### [Industrial Applicability]

The method for identifying brewer's yeast genes, the DNA array, the method of selecting brewer's yeast and the process for production of alcoholic beverages according to the invention can be utilized for production of alcoholic beverages that employ yeast fermentation.

## Claims

1. A method for identifying a brewer's yeast gene associated with alcoholic beverage flavor, the method comprising a step of selecting a factor that can affect alcoholic beverage flavor from among brewer's yeast strains or fermentation conditions and carrying out fermentation based on the selected factor to obtain a pair of fermenting brewer's yeasts for comparative contrast, and a step of comparing the pair of fermenting brewer's yeasts and identifying a gene (G1) having different levels of mRNA expression, wherein identification of the G1 gene is accomplished by a method comprising:
(1) a step of extracting total RNA from both of the pair of fermenting brewer's yeasts,
(2) a step of obtaining cDNA or cRNA labeled with a first fluorescent dye from one of the total RNAs and obtaining cDNA or cRNA labeled with a second fluorescent dye from the other of the total RNAs,
(3) a step of competitive hybridization of the cDNA labeled with the first fluorescent dye and the cDNA labeled with the second fluorescent dye, or the cRNA labeled with the first fluorescent dye and the cRNA labeled with the second fluorescent dye, on a DNA array binding plasmids containing genomic DNA fragments of the same or different brewer's yeast as the aforementioned brewer's yeast, on a plurality of spots on a substrate,
(4) a step of measuring the fluorescence emitted by the first fluorescent dye and the fluorescence emitted by the second fluorescent dye at each spot,
(5) a step of comparing the intensity of fluorescence emitted by the first fluorescent dye with the intensity of fluorescence emitted by the second fluorescent dye, and
(6) a step of identifying the G1 gene from the sequence of genomic DNA fragments in the plasmids bound at spots where a difference in fluorescence intensity is observed.

2. A method for identifying a brewer's yeast gene associated with alcoholic beverage flavor, the method comprising a step of selecting a factor that can affect alcoholic beverage flavor from among brewer's yeast strains or fermentation conditions and carrying out fermentation based on the selected factor to obtain a pair of fermenting brewer's yeasts, a step of comparing the pair of fermenting brewer's yeasts and identifying genes (G1) having different levels of mRNA expression and genes (G2) having different levels of protein expression, and a step of selecting common genes from among the identified G1 and G2 genes,
wherein identification of the G1 genes is accomplished by a method comprising:
(1) a step of extracting total RNA from both of the pair of fermenting brewer's yeasts,
(2) a step of obtaining cDNA or cRNA labeled with a first fluorescent dye from one of the total RNAs and obtaining cDNA or cRNA labeled with a second fluorescent dye from the other of the total RNAs,
(3) a step of competitive hybridization of the cDNA labeled with the first fluorescent dye and the cDNA labeled with the second fluorescent dye, or the cRNA labeled with the first fluorescent dye and the cRNA labeled with the second fluorescent dye, on a DNA array binding plasmids containing genomic DNA fragments of the same or different brewer's yeast as the aforementioned brewer's yeast, on a plurality of spots on a substrate,
(4) a step of measuring the fluorescence emitted by the first fluorescent dye and the fluorescence emitted by the second fluorescent dye at each spot,
(5) a step of comparing the intensity of fluorescence emitted by the first fluorescent dye with the intensity of fluorescence emitted by the second fluorescent dye, and
(6) a step of identifying the G1 genes from the sequence of genomic DNA fragments in the plasmids bound at spots where a difference in fluorescence intensity is observed,
and wherein identification of the G2 genes is accomplished by a method comprising:
(i) a step of extracting crude protein from both of the pair of fermenting brewer's yeasts,
(ii) a step of obtaining crude protein labeled with a third fluorescent dye from one of the crude proteins and crude protein labeled with a fourth fluorescent dye from the other of the crude proteins,
(iii) a step of separating a mixture of the crude protein labeled with the third fluorescent dye and the crude protein labeled with the fourth fluorescent dye using two-dimensional electrophoresis,
(iv) a step of measuring the fluorescence emitted by the third fluorescent dye and the fluorescence emitted by the fourth fluorescent dye binding to the fractionated protein,
(v) a step of comparing the intensity of fluorescence emitted by the third fluorescent dye with the intensity of fluorescence emitted by the fourth fluorescent dye, and
(vi) a step of analyzing the proteins exhibiting a difference in fluorescence intensity to identify G2 genes coding for the protein.

3. A method according to claim 1 or 2, wherein the DNA array used in step (3) further comprises another spot binding a brewer's yeast gene of known function.

4. A method according to claim 3, wherein in the DNA array used in step (3), blocks consisting of a plurality of spots of brewer's yeast binding plasmids including genomic DNA fragments and spots binding the brewer's yeast gene of known function, are arranged on a substrate.

5. A method according to claim 4, wherein in the DNA array used in step (3), the blocks have equal shapes, and the spots binding the brewer's yeast gene of known function are situated at the same position of each block.

6. A method according to any one of claims 1 to 5, wherein the brewer's yeast in step (3) is bottom beer yeast.

7. A DNA array wherein a gene associated with alcoholic beverage flavor that has been identified by a method according to any one of claims 1 to 6 is bound at a plurality of spots on a substrate.

8. A method for selecting brewer's yeasts based on flavor, the method comprising:
(a) a step of extracting total RNA from fermenting brewer's yeast,
(b) a step of obtaining cDNA or cRNA labeled with a fluorescent dye from the total RNA,
(c) a step of hybridizing the fluorescent dye-labeled cDNA or cRNA onto a DNA array according to claim 7,
(d) a step of measuring the fluorescence emitted by the fluorescent dye at each spot of the DNA array and
(e) a step of evaluating gene expression of the fermenting brewer's yeast based on the intensity of fluorescence of the fluorescent dye at each spot.

9. A process for production of alcoholic beverages using brewer's yeast selected by a method according to claim 8.

10. An alcoholic beverage produced by a process according to claim 9.


### [Sequence Listing]

SEQUENCE LISTING

<110>  Sapporo Breweries Ltd.

<120>  METHOD OF CONTROLLING THE FLAVOR OF ALCOHOLIC DRINK

<130>  EP53942HV163pau

<140>  not yet assigned
<141>  2006-02-27

<150>  PCT/JP2006/303646
<151>  2006-02-27

<150>  JP 2005-059716
<151>  2005-03-03

<160>  24

<170>  PatentIn version 3.1

<210>  1
<211>  20
<212>  DNA
<213>  Artificial

<220>
<223>  primer for ACT1

<400>  1
tcggtagacc aagacaccaa                                                    20


<210>  2
<211>  20
<212>  DNA
<213>  Artificial

<220>
<223>  primer for ACT1

<400>  2
ccttacggac atcgacatca                                                    20


<210>  3
<211>  20
<212>  DNA
<213>  Artificial

<220>
<223>  primer for ATF1

<400>  3
gccacatcca gtgcatgatt                                                    20


<210>  4
<211>  20
<212>  DNA
<213>  Artificial

<220>
<223>  primer for ATF1

<400>  4
tagttgtgag cggcaatctg                                                    20

<210> 5
<211> 20
<212> DNA
<213> Artificial

<220>
<223> primer for ATF2

<400> 5
cgaagaggcc taattggaga 20

<210> 6
<211> 20
<212> DNA
<213> Artificial

<220>
<223> primer for ATF2

<400> 6
tcaccgttgt cgtacgattc 20

<210> 7
<211> 20
<212> DNA
<213> Artificial

<220>
<223> primer for Lg-ATF1

<400> 7
ggtgtgattc tcaacgagca 20

<210> 8
<211> 20
<212> DNA
<213> Artificial

<220>
<223> primer for Lg-ATF1

<400> 8
aacggagtga tggtgcactt 20

<210> 9
<211> 20
<212> DNA
<213> Artificial

<220>
<223> primer for EHT1

<400> 9
taccagaggt tgtgcacgtt 20

<210> 10
<211> 20
<212> DNA
<213> Artificial

<220>
<223> primer for EHT1

<400> 10

tctgcaattg ccttggtagc                                                    20

<210>  11
<211>  20
<212>  DNA
<213>  Artificial

<220>
<223>  primer for IAH1

<400>  11
gatcagtatg ctcttggagc                                                    20

<210>  12
<211>  20
<212>  DNA
<213>  Artificial

<220>
<223>  primer for IAH1

<400>  12
gttgttgcca agcatcacca                                                    20

<210>  13
<211>  20
<212>  DNA
<213>  Artificial

<220>
<223>  primer for LEU4

<400>  13
acggtggaag cattaacagg                                                    20

<210>  14
<211>  20
<212>  DNA
<213>  Artificial

<220>
<223>  primer for LEU4

<400>  14
ggatccaatg gcaagtatgg                                                    20

<210>  15
<211>  20
<212>  DNA
<213>  Artificial

<220>
<223>  primer for OLE1

<400>  15
ctccgttttc tactacgctg                                                    20

<210>  16
<211>  20
<212>  DNA
<213>  Artificial

<220>

<223>  primer for OLE1

<400>  16
gttgggtcgt attggtacca                                                    20


<210>  17
<211>  20
<212>  DNA
<213>  Artificial

<220>
<223>  primer for SSU1

<400>  17
tgctcttacg aggcagtttg                                                    20


<210>  18
<211>  20
<212>  DNA
<213>  Artificial

<220>
<223>  primer for SSU1

<400>  18
atggcatgca gccacgttaa                                                    20


<210>  19
<211>  20
<212>  DNA
<213>  Artificial

<220>
<223>  primer for Lg-FLO1

<400>  19
caacaaagca aaccaagggg                                                    20


<210>  20
<211>  20
<212>  DNA
<213>  Artificial

<220>
<223>  primer for Lg-FLO1

<400>  20
ttaccatacg attgccagca                                                    20


<210>  21
<211>  18
<212>  DNA
<213>  Artificial

<220>
<223>  primer for 18S rRNA

<220>
<221>  misc_feature
<222>  (10)..(10)
<223>  y = t/u or c

<400>  21
ctggttgaty ctgccagt                                                      18

<210>  22
<211>  18
<212>  DNA
<213>  Artificial

<220>
<223>  primer for 18S rRNA

<220>
<221>  misc_feature
<222>  (2)..(2)
<223>  y = t/u or c

<220>
<221>  misc_feature
<222>  (10)..(10)
<223>  r = g or a

<400>  22
cygcaggttc acctacrg                                                    18


<210>  23
<211>  19
<212>  DNA
<213>  Artificial

<220>
<223>  primer for 28S rRNA

<220>
<221>  misc_feature
<222>  (1)..(1)
<223>  r = g or a

<220>
<221>  misc_feature
<222>  (17)..(17)
<223>  y = t/u or c

<220>
<221>  misc_feature
<222>  (18)..(18)
<223>  w = a or t/u

<400>  23
rcatcgatga agaacgywg                                                   19


<210>  24
<211>  22
<212>  DNA
<213>  Artificial

<220>
<223>  primer for 28S rRNA

<220>
<221>  misc_feature
<222>  (1)..(1)
<223>  m ≐ a or c

<220>
<221>  misc_feature
<222>  (2)..(2)
<223>  r = g or a

<220>
<221>  misc_feature
<222>  (7)..(7)
<223>  k = g or t/u

<220>
<221>  misc_feature
<222>  (15)..(15)
<223>  r = g or a

<220>
<221>  misc_feature
<222>  (16)..(16)
<223>  y = t/u or c

<220>
<221>  misc_feature
<222>  (17)..(17)
<223>  r = g or a

<400>  24
mrggctkaat ctcaryrgat cg                                                22